(19) Europäisches Patentamt
European Patent Office
Office européen des brevets

(11) Publication number: 0 134 633
B1

(12) EUROPEAN PATENT SPECIFICATION

(45) Date of publication of patent specification: 08.06.88

(51) Int. Cl.[4]: G 03 C 1/72 // C07D498/10
,(C07D498/10, 265:00, 209:00)

(21) Application number: 84304153.4

(22) Date of filing: 20.06.84

(54) **Photochromic composition.**

(30) Priority: 08.08.83 US 521309

(43) Date of publication of application:
20.03.85 Bulletin 85/12

(45) Publication of the grant of the patent:
08.06.88 Bulletin 88/23

(84) Designated Contracting States:
AT BE CH DE FR GB IT LI LU NL SE

(58) References cited:
GB-A-2 029 410

(73) Proprietor: AMERICAN OPTICAL CORPORATION
14 Mechanic Street
Southbridge, MA 01550 (US)

(72) Inventor: Chu, Nori Y. C.
24 Browning Road
Shrewbury Massachusetts 01545 (US)

(74) Representative: Coxon, Philip et al
Eric Potter & Clarkson 14 Oxford Street
Nottingham NG1 5BP (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 134 633 B1

**Description**

The invention relates to a photochromic composition, and more particularly to an organic photochromic composition comprising spiro (indoline-2,3'-(3*H*)-naphth-(2,1-*b*)(1,4) oxazine)(SO) dye, with substitution of a trifluoromethyl group in the indoline part of the molecule.

This application is related to US—A—4440672, published 3.4.84, entitled Photochromic Composition Resistant to Fatigue.

As used throughout this specification and claims, the terms (SO) dye, (SO) compound or (SO) derivative are intended to represent a compound having the following structural formula:

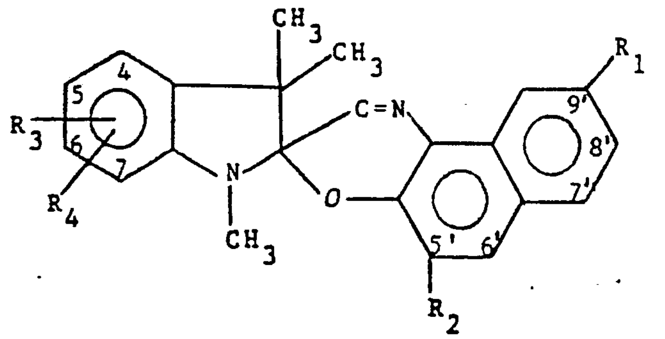

wherein "SO" is an abbreviation for spiro (indoline-2,3'-(3*H*)-naphth-(2,1-*b*)(1,4)oxazine), and hereinafter the parent compound is a (SO) compound wherein $R_1$, $R_2$, $R_3$ and $R_4$ are hydrogen.

Compounds which undergo reversible photo-induced color changes are termed photochromic compounds. When subjected to ultraviolet light or visible irradiation, these photochromic compounds change their transmission. They subsequently revert to their original color state when they are subjected to a different wavelength of radiation or when the initial light source is removed.

The photochromism of (SO) compounds was first disclosed in U.S. Patent Nos. 3,562,172 and 3,578,602. These compounds are (SO) derivatives with substitutions in the indoline part of the molecule. Subsequently, U.S. Patent Nos. 4,215,010 and 4,342,668 disclosed (SO) derivative compounds with substitution in the naphthalene part of the molecule in addition to substitution in the indoline part of the molecule. These (SO) derivative compounds in the two latter patents show an enhanced photocolorability when compared to the (SO) derivative compounds disclosed in U.S. Patent Nos. 3,562,172 and 3,578,602.

A solution of all (SO) derivative compounds in common organic solvents, as disclosed in the four above-mentioned patents, is colorless or slightly blue before ultraviolet light irradiation and becomes an intense blue color after irradiation. When (SO) derivatives are incorporated into a plastic host material, such as cellulose acetate butyrate, (CAB), the resulting material shows this same photochromic behavior. A great deal of effort has been made to avoid this intense blue color. However, until the present invention, this effort has failed as evidenced by the intense blue color shown in all of the compounds disclosed in the above-mentioned patents.

It was also found that with some of the (SO) derivative compounds of the above-mentioned patents there is a large thermal equilibration toward the colored form. Thus, when these particular (SO) compounds are incorporated into a plastic host, the resulting structure exhibits a low luminous transmittance in the unactivated state (before ultraviolet light irradiation), which renders these compounds undesirable for applications such as in lenses which go from a clear to a colored form. When an ultraviolet stabilizer is used with these (SO) derivative compounds, as disclosed in US—A—4440672, published 3.4.84 the light fatigue resistance of the (SO) dye is improved without hindering the photocolorability of the photochromic composition. Improvement of the light fatigue resistance is desirable in order to broaden the use of a photochromic article and to increase its useful lifetime.

Accordingly, it is a principal object of the present invention to provide a new class of photochromic (SO) derivative compounds which are more neutral in color after being subjected to ultraviolet light irradiation.

It is another object of the present invention to provide a new class of photochromic (SO) derivative compounds, which when incorporated into a plastic host, will produce a photochromic material with a high luminous transmittance in the unactivated state, yet which can be darkened significantly when subjected to ultraviolet light irradiation.

Yet another object is to improve the light fatigue resistance of photochromic (SO) derivative compounds of the present invention without hindering their photocolorability, by employing an ultraviolet stabilizer.

It is a further object of the present invention to incorporate such improved photochromic (SO) derivative compositions into host materials and to fabricate photochromic articles such as sunglasses, ophthalmic lenses, ski goggles, window coatings, and the like, from the resulting photochromic material.

The problems of the prior art are overcome by the discovery of a novel class of organic photochromic compounds which maintain a more neutral color when subjected to ultraviolet light irradiation. These compounds are different from prior art (SO) compounds in that there is a substitution of a trifluoromethyl group in the indoline portion of the (SO) molecule.

When incorporated into a plastic host, such as cellulose acetate butyrate (CAB), the organic photochromic compounds of the present invention produce a plastic material which exhibits a high luminous transmittance in the unactivated state (before ultraviolet light irradiation), and which can be darkened significantly when subjected to ultraviolet light.

When an ultraviolet stabilizer is used with the (SO) dyes of the present invention, such as disclosed in US—A—4440672, published 3.4.84, the light fatigue resistance of the photochromic composition is improved without hindering its photocolorability. By improving the light fatigue resistance, the useful life of the organic photochromic composition is also improved.

The organic photochromic compositions of the present invention may be incorporated within optically clear plastics to make a photochromic element suitable for photochromic sunglass lenses, ski goggles, ophthalmic lenses, window coatings, and the like. They may also be incorporated into opaque plastic materials suitable for use in toys and other articles to impart photochromic properties in such articles.

Reference is now made to the accompanying drawings, in which:

FIG. 1 illustrates the transmission spectrum of 4- and 6-trifluoromethyl-1,3,3-trimethyl-9′-methoxy (SO) and the parent compound in CAB before and after ultraviolet light irradiation; and

FIG. 2 illustrates the transmission spectrum of 4- and 6-trifluoromethyl 1,3,3-trimethyl-5′-methoxy (SO) and the parent compound in CAB before and after ultraviolet light irradiation.

At the outset the invention is described in its broadest overall aspects with a more detailed description following.

The organic photochromic composition of the present invention comprises spiro(indoline-2,3′-(3*H*)-naphth-(2,1-*b*)(1,4)oxazine) (SO) dye, of the formula

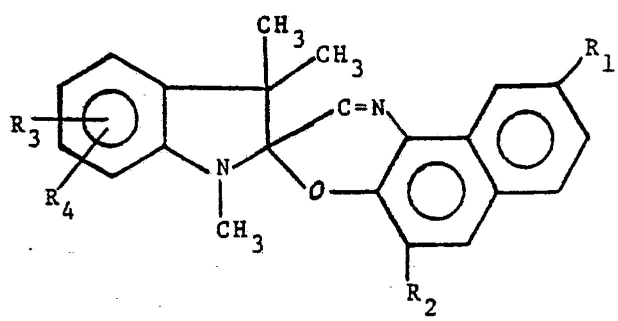

wherein one of $R_1$ and $R_2$ is a lower alkoxy group and the other is hydrogen; $R_3$ is a trifluoromethyl group; and $R_4$ is hydrogen, a lower alkyl or a lower alkoxy group. $R_3$ and $R_4$ are independently located at the 4, 5, 6, or 7 position of the indoline ring, although, of course, there can be only one substituent at any one position. By lower alkyl or lower alkoxy is meant a moiety containing 1 to 4 carbon atoms.

When the organic photochromic composition of the present invention is to be incorporated into a plastic host, the preferred class of compounds comprises a lower alkoxy group at $R_1$ or $R_2$ and most preferably methoxy or ethoxy, trifluoromethyl at $R_3$, and hydrogen or methyl at $R_4$. This gives the resulting photochromic plastic material a high luminous transmittance in the unactivated state (before ultraviolet irradiation), which can be darkened significantly in the presence of ultraviolet light irradiation.

In accordance with the present invention, the preferred (SO) dyes are:

4-trifluoromethyl-1,3,3-trimethyl-5′-methoxy spiro (indoline-2,3′-(3*H*)-naphth-(2,1-*b*)(1,4)oxazine);
6-trifluoromethyl-1,3,3-trimethyl-5′-methoxy spiro (indoline-2,3′-(3*H*)-naphth-(2,1-*b*)(1,4)oxazine);
4-trifluoromethyl-1,3,3-trimethyl-9′-methoxy spiro (indoline-2,3′-(3*H*)-naphth-(2,1-*b*)(1,4)oxazine);
6-trifluoromethyl-1,3,3-trimethyl-9′-methoxy spiro (indoline-2,3′-(3*H*)-naphth-(2,1-*b*)(1,4)oxazine);
5-trifluoromethyl-1,3,3-trimethyl-9′-methoxy spiro (indoline-2,3′-(3*H*)-naphth-(2,1-*b*)(1,4)oxazine);
$Y_1$-trifluoromethyl-$Y_2$-methyl-1,3,3-trimethyl-9′-methoxy spiro (indoline-2,3′-(3*H*)-naphth-(2,1-*b*)(1,4)oxazine);
4-trifluoromethyl-1,3,3-trimethyl-5′-ethoxy spiro (indoline-2,3′-(3*H*)-naphth-(2,1-*b*)(1,4)oxazine);
6-trifluoromethyl-1,3,3-trimethyl-5′-ethoxy spiro (indoline-2,3′-(3*H*)-naphth-(2,1-*b*)(1,4)oxazine);
4-trifluoromethyl-1,3,3-trimethyl-9′-ethoxy spiro (indoline-2,3′-(3*H*)-naphth-(2,1-*b*)(1,4)oxazine);
6-trifluoromethyl-1,3,3-trimethyl-9′-ethoxy spiro (indoline-2,3′-(3*H*)-naphth-(2,1-*b*)(1,4)oxazine);
$Y_1$-trifluoromethyl-$Y_2$-methyl-1,3,3-trimethyl-5′-methoxy spiro (indoline-2,3′-(3*H*)-naphth-(2,1-*b*)(1,4)oxazine).

Reference to $Y_1$ and $Y_2$ indicates attachment at the 4, 5, 6 or 7 position, although both cannot be at the same position, but the exact position was not determined.

To enhance the light fatigue resistance of the organic photochromic material of the present invention, without affecting its photocolorability, it is preferable to add an ultraviolet stabilizer beloging to the class of

peroxide decomposers or the class of excited state quenchers, preferably a singlet oxygen quencher, and most preferably a complex of $Ni^{2+}$ ion with an organic ligand. These $Ni^{2+}$ complexes are normally used in polyolefins to provide protection from photodegradation. The ultraviolet stabilizers used in the present invention will not hinder the photocolorability of the (SO) dyes, because they have a minimal absorption in the ultraviolet region where (SO) dyes absorb. Improving the light fatigue resistance also improves the useful life of the organic photochromic compositions of the present invention.

Preferably, between 0.01 and about 15% by weight of the (SO) dye and between 0.1 and about 5% by weight of the ultraviolet stabilizer, depending on its solubility, is incorporated into an optically clear plastic material having enhanced light fatigue resistance. The optically clear matrix will preferably have a thickness in the range of 0.000254 —5.08 cm (0.0001—2.0 inch). As used throughout this specification and claims, all percentages are by weight unless otherwise specified.

In another important embodiment, the (SO) dye and ultraviolet stabilizer is mixed in solution with an optically clear pre-polymer liquid which is thereafter cast as a film or lens, or otherwise incorporated into a polymer which is injection molded or shaped into a film or lens; or a prepolymerized film or lens containing the ultraviolet stabilizer may be immersed in a dye bath comprising (SO) dye dissolved in a solution of organic solvents such as alcohol, toluene, halogenated hydrocarbon or the like. Other methods of blending the ultraviolet stabilizer with the (SO) dye and optically clear polymer, such as coating or laminating may also be employed.

Ultraviolet stabilizers useful herein include complexes of $Ni^{2+}$ ion with some organic ligand, cobalt (III) tri-di-n-butyldithiocarbamate, and cobalt (II) di-iso-propyldithiocarbamate.

The preferred ultraviolet stabilizers are $Ni^{2+}$ complexes and more particularly (2,2′-thiobis(4-(1,1,3,3-tetramethylbutyl) phenolato)(butylamine)nickel

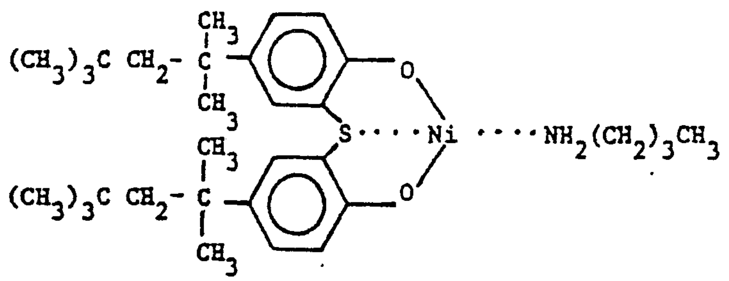

sold under the tradename of Cyasorb UV 1084, obtained from the American Cyanamid Company; nickel (O-ethyl(3,5-di-tert-butyl-4-hydroxybenzyl) phosphonate

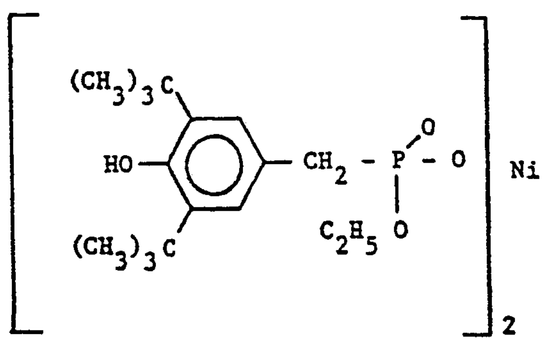

sold under the tradename of Irgastab 2002, obtained from the Ciba-Geigy Corporation; nickel dibutyldithiocarbomate

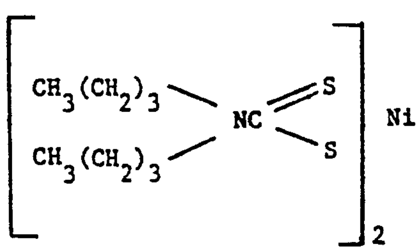

sold under the tradename of Rylex NBC, obtained from E. I. du Pont de Nemours & Company; bis (2,2′-thiobis-4-(1,1,3,3-tetramethybutyl) phenolato) nickel

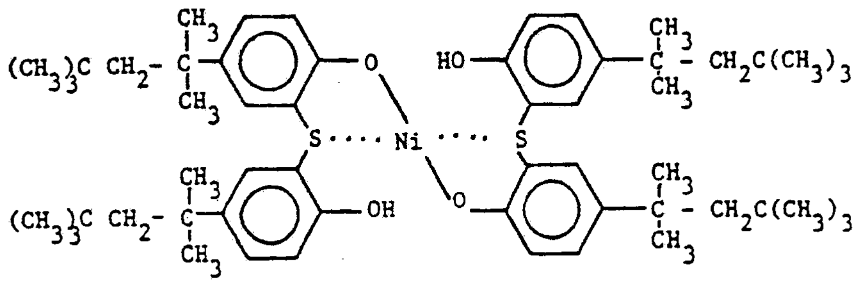

sold under the tradename of UV-Chek AM 101, obtained from the Ferro Corporation; nickel di-isopropyldithiophosphate (NIDIPDTP);

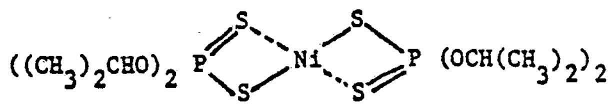

and other $Ni^{2+}$ complexes sold under the tradenames of UV-Chek AM 105, UV-Chek AM 126, and UV-Chek AM 205 which can also be obtained from the Ferro Corporation.

The preferred transparent plastic hosts are cellulose acetate butyrate (CAB); CR—39[tm], a diethylene glycol bis(allylcarbonate) obtained from PPG Industries, Inc.; Lexan[tm], a polycarbonate condensation product of bisphenol-A and phosgene, obtained from General Electric; and Plexiglas[tm], a polymethyl methacrylate obtained from the Rohm and Haas Company. Other plastic materials into which the photochromic compositions of this invention may be incorporated include polyvinyl chloride, polyvinyl acetate, polystyrene, polyurethane, polyesters and epoxies. The invention is further illustrated by the following non-limiting examples (as used throughout the examples, the formula $CF_3$ is intended to represent trifluoromethyl; the formula $CH_3O$ is intended to represent methoxy; and the formula $C_2H_5O$ is intended to represent ethoxy).

Examples 1—5

A mixture of 22 grams of 1-nitroso-7-methoxy-2-naphthol in 250 ml. of ethanol was bubbled with $N_2$ gas for 15 minutes before bringing it to a gentle reflux. A solution of 36.9 grams of a mixture of 4- and 6-trifluoromethyl-1,2,3,3-tetramethyl-indoleninium iodide isomers in 50 ml. of ethanol containing 12 grams of triethylamine was added over a period of 30 minutes. After the mixture was refluxed for two hours, the solution was then cooled and the resulting brown precipitate was separated by filtering. The crude product was purified by recrystallization from acetone. A set of photochromic compounds was synthesized in this manner using starting materials having the appropriate substituents. A list of these compounds is shown in Table I.

TABLE I

List of Compounds Synthesized

| | |
|---|---|
| 1. $R_1 = CH_3O$<br>$R_2 = H$<br>$R_3 = 4$ & $6\text{-}CF_3$ (mixture)<br>$R_4 = H$ | 2. $R_1 = CH_3O$<br>$R_2 = H$<br>$R_3 = 5\text{-}CF_3$<br>$R_4 = H$ |
| 3. $R_1 = C_2H_5O$<br>$R_2 = H$<br>$R_3 = 4$ & $6\text{-}CF_3$ (mixture)<br>$R_4 = H$ | 4. $R_1 = H$<br>$R_2 = CH_3O$<br>$R_3 = 4$ & $6\text{-}CF_3$ (mixture)<br>$R_4 = H$ |
| 5. $R_1 = H$<br>$R_2 = C_2H_5O$<br>$R_3 = 4$ & $6\text{-}CF_3$ (mixture)<br>$R_4 = H$ | |

Example 6—7

Benzotrifluoride (29 gm) was added to a solution of butyl lithium (13.5 gm) in ether (250 ml) which was cooled by an ice-water bath, then this mixture was refluxed for 2 hours. After cooling, a solution of methyl iodide (35 gm) in ether (100 ml) was added slowly over a period of one hour. Subsequently, the mixture was refluxed for 8 hours and then distilled, with the fraction boiling in the range of 120—128°C collected. The yield was 19 gm of methyl-benzotrifluoride isomers.

To the methylbenzotrifluoride isomers (16 gm) cooled in an ice-water bath was slowly added a solution of concentrated nitric acid (20 ml) and concentrated sulfuric acid (20 ml) to keep $T \leq 55$°C. The mixture was then stirred for 0.5 hours at 50°C., followed by vacuum distillation and collected at 105—130°C.

The nitro-methylbenzotrifluoride isomer mixture (15 gm) in ethanol (300 ml) was hydrogenated at 20—45°C and 276 kPa (40 psi) over Pd-on-carbon catalyst (0.1 gm) for 5—8 hours. The mixture was then distilled to give a mixture of amino-methylbenzotrifluoride isomers.

The desired indolenium iodide isomers were obtained by the well known Fischer indole synthesis and the final (SO) dye was obtained as described in the Examples 1—5. The dyes prepared had the following substituent selection, wherein $Y_1$ and $Y_2$ indicate attachment at the 4, 5, 6 or 7 position, but the exact position of attachment was not determined.

| | |
|---|---|
| 6. $R_1 = CH_3O$<br>$R_2 = H$<br>$R_3 = Y_1—CF_3$<br>$R_4 = Y_2—CH_3$ | 7. $R_1 = O$<br>$R_2 = CH_3O$<br>$R_3 = Y_1—CF_3$<br>$R_4 = Y_2—CH_3$ |

Example 8—14

A series of CAB films were cast in the thickness of 0.00889 to 0.01016 cm (4.0 mils) and contained two weight percent of photochromic compounds prepared in accordance with Examples 1—7. The films were colorless, but became an intense purplish-gray color upon exposure to ultraviolet light irradiation, in contrast to the deep blue color shown by the prior art compounds.

The positions of the main absorption peaks in the visible region of the spectrum of these CAB films after ultraviolet light irradiation are shown in Table II, which illustrates the substantial spectral shift induced by the substitution of a trifluoromethyl group in the indoline part of the molecule.

Table II

Position of the Absorption Peaks of the (SO) Compounds with a Trifluoromethyl Substituent

| Compounds | Positions (nm) |
|---|---|
| 4- & 6-$CF_3$-9'-$CH_3O$ | 590, 570, 490 |
| 4- & 6-$CF_3$-5'-$CH_3O$ | 570, 490 |
| 5-$CF_3$-9'-$CH_3O$ | 600, 570 490 |
| 4- & 6-$CF_3$-9'-$C_2H_5O$ | 600, 570, 490 |
| 4- & 6-$CF_3$-5'-$C_2H$ | 575, 490 |
| $Y_1$—$CF_3$—$Y_2$—$CH_3$-9'-$CH_3O$ | 580, 485 |
| $Y_1$—$CF_3$—$Y_2$—$CH_3$-9'-$CH_3O$ | 600, 575, 490 |
| ($R_1$—$R_4$ = H) | 610, 570 (SH)* |

* Shoulder (not a complete peak)

As a further illustration, the activated spectrum of 4- and 6-trifluoromethyl-9'-methoxy, and 4- and 6-trifluoromethyl-5'-methoxy (SO) dyes and of the parent compound without any substituent is shown in Figs. 1 and 2 to demonstrate this large spectral shift induced by a trifluoromethyl substituent. Curves 1 and 2 are for the trifluoromethyl derivative and Curves 3 and 4 are for the parent compound ($R_1$, $R_2$, $R_3$ and $R_4$ = H).

Examples 15—21

A series of CAB films was cast in accordance with Examples 8—14. The luminous transmittance (L.T.), in percent, of the unactivated (before ultraviolet light irradiation) films was measured and is shown in Table III. The data clearly indicates that the trifluoromethyl group greatly suppresses the initial coloration of 5'-methoxy derivative compounds and, hence, increases the usefulness of this particular class of (SO) derivatives.

Table III

Luminous Transmittance in Unactivated State

| Substituent(s) in Indoline | L.T. (%) |
|---|---|
| H (compound of prior art) | 67 |
| 5-$CH_3$ (compound of prior art) | 50 |
| 5-$CH_3O$ (compound of prior art) | 40 |
| 5-CL (compound of prior art) | 79 |
| 4- & 6-$CF_3$ (mixture) | 90 |
| $Y_1$—$CF_3$—$Y_2$—$CH_3$ | 82 |
| 4- & 6-$CF_3$-5'-$C_2H_5O$ | 87 |

Examples 22—26

As set of CAB films was cast from a 50 gram solution of 10% CAB in methylene chloride containing 100 mgs. 4- and 6-trifluoromethyl-9'-methoxy (SO) isomer mixture and 50 mgs. of ultravioet stabilizer $Ni^{2+}$ complexes (13 mgs of Rylex NBC). The $Ni^{2+}$ complexes used were (2,2'-thiobis(4-(1,1,3,3-tetramethylbutyl)-phenolato) (butylamine) nickel (sold under the tradename of Cyasorb UV 1084 and obtained from the American Cyanamid Company a $Ni^{2+}$ complex sold under the tradename of UV-Chek AM—105 and

obtained from the Ferro corporation; a $Ni^{2+}$ complex sold under the tradename of UV-Chek AM—126 and obtained from the Ferro Corporation; and nickel dibutyldithiocarbamate (sold under the tradename of Rylex NBC and obtained from E. I. du Pont de Nemours & Company. A control without the $Ni^{2+}$ complexes was also cast.

These five CAB films were subjected to 20-hour cycle exposures in a Fadeometer manufactured by Atlas Electric Devices of Chicago, Illinois. After three 20-hour cycles of Fadeometer exposure, the control lost all of its photochromism. As shown in Table IV, the CAB films compounded with the $Ni^{2+}$ complexes still showed good photochromism.

TABLE IV

Residual Photocolorability of CAB Films after 60 Hours of Fadeometer Exposure

| Compound | Residual Photocolorability (%) |
|---|---|
| Cyasorb UV 1084 | 87 |
| UV-Chek AM—105 | 78 |
| UV-Chek AM—126 | 76 |
| Rylex NBC | 95 |
| Control | 0 |

While the invention has been described with reference to its preferred embodiment, other embodiments can achieve the same results.

## Claims

1. An organic photochromic composition comprising at least one photochromic compound, characterised in that said photochromic compound has the structural formula:

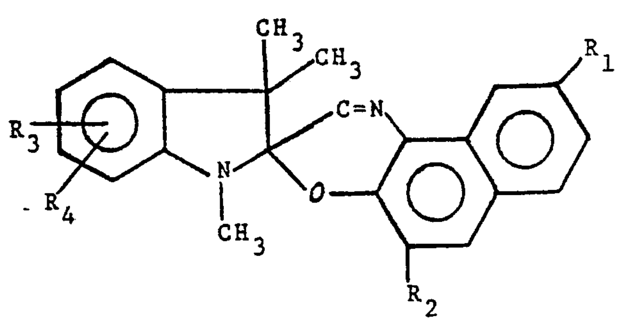

wherein one of $R_1$ and $R_2$ is an alkoxy group having 1 to 4 carbon atoms and the other is hydrogen; $R_3$ is a trifluoromethyl group; and $R_4$ is hydrogen, an alkyl group having 1 to 4 carbon atoms or an alkoxy group having 1 to 4 carbon atoms.

2. The composition of Claim 1 further comprising an ultraviolet stabilizer belonging to the class of peroxide decomposers or the class of excited state quenchers.

3. The composition of Claim 2 wherein the ultraviolet stabilizer comprises a singlet oxygen quencher such as cobalt (II) di-iso-propyldithiocarbamate or a complex of $Ni^{2+}$ ion with an organic ligand.

4. The composition of Claim 3 wherein the $Ni^{2+}$ complex comprises (2,2'-thiobis(4-1,1,3,3-tetramethylbutyl) phenolato) (butylamine) nickel; nickel (O-ethyl (3,5-di-tert-butyl-4-hydroxybenzyl)) phosphonate; nickel dibutyldithiocarbamate; bis(2,2'-thiobis-4-(1,1,3,3-tetramethylbutyl)phenolato) nickel; or nickel di-isopropyldithiophosphate.

5. The composition of any of Claims 1 to 4 wherein $R_1$ is methoxy or ethoxy, $R_2$ is hydrogen, $R_3$ is trifluoromethyl and $R_4$ is hydrogen.

6. The composition of any of Claims 1 to 4 wherein $R_1$ is hydrogen, $R_2$ is methoxy or ethoxy, $R_3$ is trifluoromethyl and $R_4$ is hydrogen.

7. The composition of any of Claims 1 to 4 wherein $R_1$ is methoxy or ethoxy, $R_2$ is hydrogen, $R_3$ is trifluoromethyl and $R_4$ is methyl.

8. The composition of any of Claims 1 to 4 wherein $R_1$ is hydrogen, $R_2$ is methoxy or ethoxy, $R_3$ is trifluoromethyl and $R_4$ is methyl.

9. The composition of any of Claims 1 to 8 wherein the photochromic compound is selected from 4-trifluoromethyl-1,3,3-trimethyl-5′-methoxy spiro (indoline-2,3′-(3*H*)-naphth-(2,1-*b*)(1,4)oxazine), 6-trifluoromethyl-1,3,3-trimethyl-5′-methoxy spiro (indoline-2,3′-(3*H*)-naphth-(2,1-*b*)(1,4)oxazine), 4-trifluoromethyl-1,3,3-trimethyl-9′-methoxy spiro (indoline-2,3′-(3*H*)-naphth-(2,1-*b*)(1,4)oxazine), 6-trifluoromethyl-1,3,3-trimethyl-9′-methoxy spiro (indoline-2,3′-(3*H*)-naphth-(2,1-*b*)(1,4)oxazine), 4-trifluoromethyl-1,3,3-trimethyl-9′-ethoxy spiro (indoline-2,3′-(3*H*)-naphth-(2,1-*b*)(1,4)oxazine), 6-trifluoromethyl 1,3,3-trimethyl-9′-ethoxy spiro (indoline-2,3′-(3*H*)-naphth-(2,1-*b*)(1,4)oxazine), 4-trifluoromethyl-1,3,3-trimethyl-5′-ethoxy spiro (indoline-2,3′-(3*H*)-naphth-(2,1-*b*)(1,4)oxazine), 6-trifluoromethyl-1,3,3-trimethyl-5′-ethoxy spiro (indoline-2,3′-(3*H*)-naphth-(2,1-*b*))(1,4)oxazine), and mixtures thereof.

10. A photochromic article comprising:

a) a plastic host; and

b) at least one photochromic composition comprising at least one photochromic compound incorporated into said host characterised in that said compound comprises the photochromic compound defined in any of Claims 1 to 9.

11. The photochromic article of Claim 10 wherein the host is cellulose acetate butyrate; polycarbonate resin; polymethyl methacrylate; or diethylene glycol bis (allyl carbonbate).

12. The photochromic article of Claim 10 or 11 in which the photochromic compound comprises a mixture of 4- and 6-trifluoromethyl 1,3,3-trimethyl-5′methoxy spiro (indoline-2,3′-(3*H*)-naphth-(2,1-*b*)(1,4)-oxazine); a mixture of 4- and 6-trifluoromethyl-1,3,3-trimethyl-9′-methoxy spiro (indoline-2,3′-(3*H*)-naphth-(2,1-*b*)(1,4) oxazine); a mixture of 4- and 6-trifluoromethyl-1,3,3-trimethyl-5′-ethoxy spiro (indoline-2,3′-(3*H*)-naphth-(2,1-*b*)(1,4)oxazine); a mixture of 4- and 6-trifluoromethyl 1,3,3-trimethyl-9′-ethoxy spiro (indoline-2,3′-(3*H*)-naphth-(2,1-*b*)(1,4)oxazine); 5-trifluoromethyl-1,3,3-trimethyl-9′-methoxy spiro (indoline-2,3′-(3*H*)-naphth-(2,1-*b*)(1,4)oxazine); $Y_1$-triofluoromethyl-$Y_2$-methyl-1,3,3-trimethyl-5′-methoxy spiro (indoline-2,3′-(3*H*)-naphth (2,1-*b*)(1,4-oxazine), wherein $Y_1$ and $Y_2$ indicates attachment at the 4, 5, 6, or 7 position; or $Y_1$-trifluoromethyl-$Y_2$-methyl-1,3,3-trimethyl-9′-methoxy spiro (indoline-2,3′-(3*H*)-naphth (2,1-*b*)(1,4)oxazine), wherein $Y_1$ and $Y_2$ indicates attachment at the 4, 5, 6 or 7 position.

13. A photochromic article according to any of Claims 10 to 12 in the form of a lens.

**Patentansprüche**

1. Organische photochrome Zusammensetzung, enthaltend mindestens eine photochrome Verbindung, dadurch gekennzeichnet, daß die photochrome Verbindung der Strukturformel entspricht:

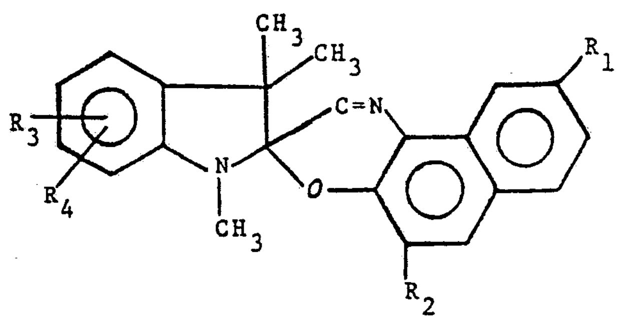

worin einer der Reste $R_1$ und $R_2$ ein Alkoxyrest mit 1 bis 4 Kohlenstoffatomen ist und der andere Wasserstoff, $R_3$ eine Trifluormethylgruppe und $R_4$ Wasserstoff, ein Alkylrest mit 1 bis 4 Kohlenstoffatomen oder ein Alkoxyrest mit 1 bis 4 Kohlenstoffatomen sind.

2. Zusammensetzung nach Anspruch 1, die weiter einen Ultraviolettlichtstabilisator enthält, der zu der Klasse der Peroxidzersetzer oder der Klasse der angeregten Zustandslöscher gehört.

3. Zusammensetzung nach Anspruch 2, worin der Ultraviolettlichtstabilisator einen Singulett-Sauertoff-Löscher enthält, wie Kobalt(II)-di-iso-propyldithiocarbamat oder einen Komplex eines $Ni^{2+}$-Ions mit einem organischen Liganden.

4. Zusammensetzung nach Anspruch 3, worin der $Ni^{2+}$-Komplex 2,2′-Thiobis(4-1,1,3,3-tetramethylbutyl)-phenolat(butylamin)-nickel, Nickel-(O-ethyl-(3,5-di-tert-butyl-4-hydroxybenzyl)-phosphonat, Nickeldibutyldithiocarbamat, Bis(2,2′-thiobis-4-(1,1,3,3-tetramethylbutyl)-phenolat)-nickel oder Nickel-diisopropyldithiophosphat enthält.

5. Zusammensetzung nach einem der Ansprüche 1 bis 4, worin $R_1$ ein Methoxy- oder Ethoxyrest, $R_2$ Wasserstoff, $R_3$ eine Trifluormethylgruppe und $R_4$ Wasserstoff sind.

6. Zusammensetzung nach einem der Ansprüche 1 bis 4, worin $R_1$ Wasserstoff, $R_2$ eine Methoxy- oder eine Ethoxygruppe, $R_3$ eine Trifluormethylgruppe und $R_4$ Wasserstoff sind.

7. Zusammensetzung nach einem der Ansprüche 1 bis 4, worin $R_1$ eine Methoxy- oder eine Ethoxygruppe, $R_2$ Wasserstoff, $R_3$ eine Trifluormethylgruppe und $R_4$ eine Methylgruppe sind.

8. Zusammensetzung nach einem der Ansprüche 1 bis 4, worin $R_1$ Wasserstoff, $R_2$ eine Methoxy- oder Ethoxygruppe, $R_3$ eine Trifluormethylgruppe und $R_4$ eine Methylgruppe sind.

9. Zusammensetzung nach einem der Ansprüche 1 bis 8, worin die photochrome Verbindung aus 4-Trifluormethyl-1,3,3-trimethyl-5'-methoxy-spiro-(indolin-2,3'-(3H)-naphth-(2,1-b)(1,4)-oxazin), 6-Trifluormethyl-1,3,3-trimethyl-5'-methoxy-spiro-(indolin-2,3'-(3H)-naphth-(2,1-b)(1,4)-oxazin), 4-Trifluormethyl-1,3,3-trimethyl-9'-methoxy-spiro-(indolin-2,3'-(3H)-naphth-(2,1-b)(1,4)-oxazin), 6-Trifluormethyl-1,3,3-trimethyl-9'-methoxy-spiro-(indolin-2,3'-(3H)-naphth-(2,1-b)(1,4)-oxazin), 4-Trifluormethyl-1,3,3-trimethyl-9'-ethoxy-spiro-(indolin-2,3'-(3H)-naphth-(2,1-b)(1,4)-oxazin), 6-Trifluormethyl-1,3,3-trimethyl-9'-ethoxy-spiro-(indolin-2,3'-(3H)-naphth-(2,1-b)(1,4)-oxazin), 4-Trifluormethyl-1,3,3-trimethyl-5'-ethoxy-spiro-(indolin-2,3'-(3H)-naphth-(2,1-b)(1,4)-oxazin), 6-Trifluormethyl-1,3,3-trimethyl-5'-ethoxy-spiro-(indolin-2,3'-(3H)-naphth-(2,1-b)(1,4)-oxazin) und Mischungen davon ausgewählt ist.

10. Photochromes Erzeugnis enthaltend

a) eine Kunstoffgrundmasse, und

b) mindestens eine photochrome Zusammensetzung, enthaltend mindestens eine photochrome Verbindung, eingearbeitet in dieses Grundmaterial, dadurch gekennzeichnet, daß diese Verbindung die photochrome Verbindung nach eiem der Ansprüche 1 bis 9 enthält.

11. Photochromes Erzeugnis nach Anspruch 10, worin die Grundmasse Zelluloseacetatbutyrat, ein Polycarbonatharz, Polymethylmethacrylat oder Diethylenglykol-bis-(allylcarbonat) ist.

12. Photochromes Erzeugnis nach Anspruch 10 oder 11, in dem die photochrome Verbindung eine Mischung aus 4- und 6-Trifluormethyl-1,3,3-trimethyl-5'-methoxy-spiro-(indolin-2,3'-(3H)-naphth-(2,1-b)-(1,4)-oxazin), eine Mischung aus 4- und 6-Trifluormethyl-1,3,3-trimethyl-9'-methoxy-spiro-(indolin-2,3'-(3H)-naphth-(2,1-b)(1,4)-oxazin), eine Mischung aus 4- und 6-Trifluormethyl-1,3,3-trimethyl-5'-ethoxy-spiro-(indolin-2,3'-(3H)-naphth-(2,1-b)(1,4)-oxazin), eine Mischung aus 4- und 6-Trifluormethyl-1,3,3-trimethyl-9'-ethoxy-spiro-(indolin-2,3'-(3H)-naphth-(2,1-b)(1,4)-oxazin), 5-Trifluormethyl-1,3,3-trimethyl-9'-methoxy-spiro-(indolin-2,3'-(3H)-naphth-(2,1-b)(1,4)-oxazin), $Y_1$-Trifluormethyl-$Y_2$-methyl-1,3,3-trimethyl-5'-methoxy-spiro-(indolin-2,3'-(3H)-naphth-(2,1-b)(1,4)-oxazin), enthält, worin $Y_1$ und $Y_2$ auf eine Anhaftung in der Position 4, 5, 6 oder 7 hinweisen, oder $Y_1$-Trifluormethyl-$Y_2$-methyl-1,3,3-trimethyl-9'-methoxy-spiro-(indolin-2,3'-(3H)-naphth-(2,1-b)(1,4)-oxazin), worin $Y_1$ und $Y_2$ auf eine Anhaftung in der Position 4, 5, 6 oder 7 hinweisen.

13. Photochromes Erzeugnis nach einem der Ansprüche 10 bis 12 in der Form einer Linse.

## Revendications

1. Composition photochromique organique renfermant au moins un composé photochromique, caractérisée en ce que ledit composé photochromique répond à la formule structurelle:

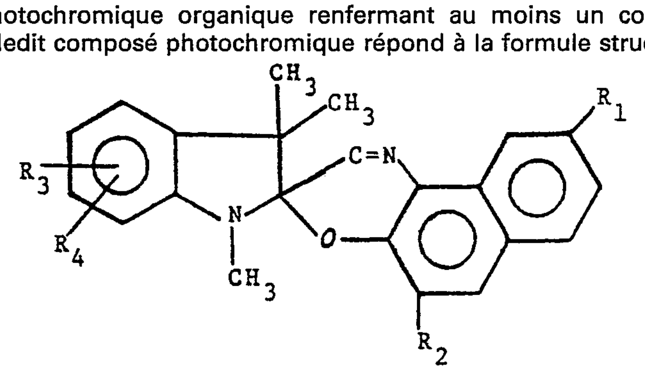

dans laquelle l'un des radicaux $R_1$ et $R_2$ est un groupe alcoxy ayant de 1 à 4 atomes de carbone et l'autre représente l'hydrogène; $R_3$ est un groupe trifluorométhyle; et $R_4$ représente l'hydrogène, un groupe alkyle ayant de 1 à 4 atomes de carbone ou un groupe alcoxy ayant de 1 à 4 atomes de carbone.

2. Composition selon la revendication 1, renfermant en outre un stabilisant vis-à-vis des ultra-violets appartenant à la classe des produits décomposant les peroxydes ou la classe des extincteurs d'état excité.

3. Composition selon la revendication 2, dans laquelle le stabilisant vis-à-vis des ultra-violets est constitué d'un extincteur de singlet oxygène tel que le di-iso-propyldithiocarbamate de cobalt (II) ou un complexe d'ion $Ni^{2+}$ avec un groupe de coordination organique.

4. Composition selon la revendication 3, dans laquelle le complexe de $Ni^{2+}$ est formé du (2,2'-thiobis(4-1,1,3,3-tétraméthyl-butyl) phénolato)(butylamine)nickel; du (O-éthyl-(3,5-di-tert-butyl-4-hydroxybenzyl)) phosphonate de nickel; du dibutyldithiocarbamate de nickel; du bis(2,2'-thiobis-4-(1,1,3,3-tétraméthylbutyl)phénolato)nickel; ou du di-isopropyldithiophosphate de nickel.

5. Composition selon l'une quelconque des revendications 1 à 4, dans laquelle $R_1$ est un groupe méthoxy ou éthoxy, $R_2$ représente l'hydrogène, $R_3$ est un groupe trifluorométhyle et $R_4$ représente l'hydrogène.

6. Composition selon l'une quelconque des revendications 1 à 4, dans laquelle $R_1$ représente l'hydrogène, $R_2$ représente un groupe méthoxy ou éthoxy, $R_3$ est un groupe trifluorométhyle et $R_4$ représente l'hydrogène.

7. Composition selon l'une quelconque des revendications 1 à 4, dans laquelle $R_1$ est un groupe méthoxy ou éthoxy, $R_2$ représente l'hydrogène, $R_3$ est un groupe trifluorométhyle et $R_4$ est un groupe méthyle.

8. Composition selon l'une quelconque des revendication 1 à 4, dans laquelle $R_1$ représente l'hydrogène, $R_2$ est un groupe méthoxy ou éthoxy, $R_3$ est un groupe trifluorométhyle et $R_4$ est un groupe méthyle.

9. Composition selon l'une quelconque des revendications 1 à 8, dans laquelle le composé photochromique est choisi parmi: la 4-trifluorométhyl-1,3,3-triméthyl-5'-méthoxy spiro (indoline-2,3'-(3*H*)-napht-(2,1-*b*)(1,4)oxazine), la 6-trifluorométhyl-1,3,3-triméthyl-5'-méthoxy spiro (indoline-2,3'-(3*H*)-napht-(2,1-*b*)-(1,4)oxazine), la 4-trifluorométhyl-1,3,3-triméthyl-9'-méthoxy spiro (indoline-2,3'-(3*H*)-napht-(2,1-*b*)(1,4)-oxazine), la 6-trifluorométhyl-1,3,3-triméthyl-9'-méthoxy spiro (indoline-2,3'-(3*H*)-napht-(2,1-*b*)(1,4)-oxazine), la 4-trifluorométhyl-1,3,3-triméthyl-9'-éthoxy spiro (indoline-2,3'-(3*H*)-napht-(2,1-*b*)(1,4)oxazine), la 6-trifluorométhyl-1,3,3-triméthyl-9'-éthoxy spiro (indoline-2,3'-(3*H*)-napht-(2,1-*b*)(1,4)oxazine), la 4-trifluorométhyl-1,3,3-triméthyl-5'-éthoxy spiro (indoline-2,3'-(3*H*)-napht-(2,1-*b*)(1,4)oxazine), la 6-trifluorométhyl-1,3,3-triméthyl-5'-éthoxy spiro (indoline-2,3'-(3*H*)-napht-(2,1-*b*)(1,4)oxazine), et les mélanges de celles-ci.

10. Article photochromique constitué:

a) d'une matière plastique d'accueil; et

b) d'au moins une composition photochromique renfermant au moins un composé photochromique incorporé dans ledit matériau d'accueil, caractérisé en ce que ledit composé est constitué du composé photochromique selon l'une quelconque des revendications 1 à 9.

11. Article photochromique selon la revendication 10, dans lequel le matériau d'accueil est l'acétobutyrate de cellulose; une résine de polycarbonate; le poly(méthacrylate de méthyle); ou le bis (allyl carbonate) de diéthylène glycol.

12. Article photochromique selon la revendication 10 ou 11, dans lequel le composé photochromique est formé d'un mélange de 4- et 6-trifluorométhyl-1,3,3-triméthyl-5'-méthoxy spiro (indoline-2,3'-(3*H*)-napht-(2,1-*b*)(1,4)oxazine); un mélange de 4- et 6-trifluorométhyl-1,3,3-triméthyl-9'-méthoxy spiro (indoline-2,3'-(3*H*)-napht-(2,1-*b*)(1,4)oxazine); un mélange de 4- et 6-trifluorométhyl-1,3,3-triméthyl-5'-éthoxy spiro (indoline-2,3'-(3*H*)-napht-(2,1-*b*)(1,4)oxazine); un mélange de 4- et 6-trifluorométhyl-1,3,3-triméthyl-9'-éthoxy spiro (indoline-2,3'-(3*H*)-napht-(2,1-*b*)(1,4)oxazine); 5-trifluorométhyl-1,3,3-triméthyl-9'-méthoxy spiro (indoline-2,3'-(3*H*)-napht-(2,1-*b*)(1,4)oxazine); $Y_1$-trifluorométhyl-$Y_2$-méthyl-1,3,3-triméthyl-5'-méthoxy spiro (indoline-2,3'-(3*H*)-napht-(2,1-*b*)(1,4)oxazine), dans laquelle $Y_1$ ou $Y_2$ indiquent une fixation en position 4, 5, 6 ou 7; ou $Y_1$-trifluorométhyl-$Y_2$-méthyl-1,3,3-triméthyl-9'-méthoxy spiro (indoline-2,3'-(3*H*)-napht-(2,1-*b*)(1,4)oxazine), dans laquelle $Y_1$ et $Y_2$ indiquent une fixation en position 4, 5, 6 ou 7.

13. Article photochromique selon l'une quelconque des revendications 10 à 12 se présentant sous la forme d'une lentille.

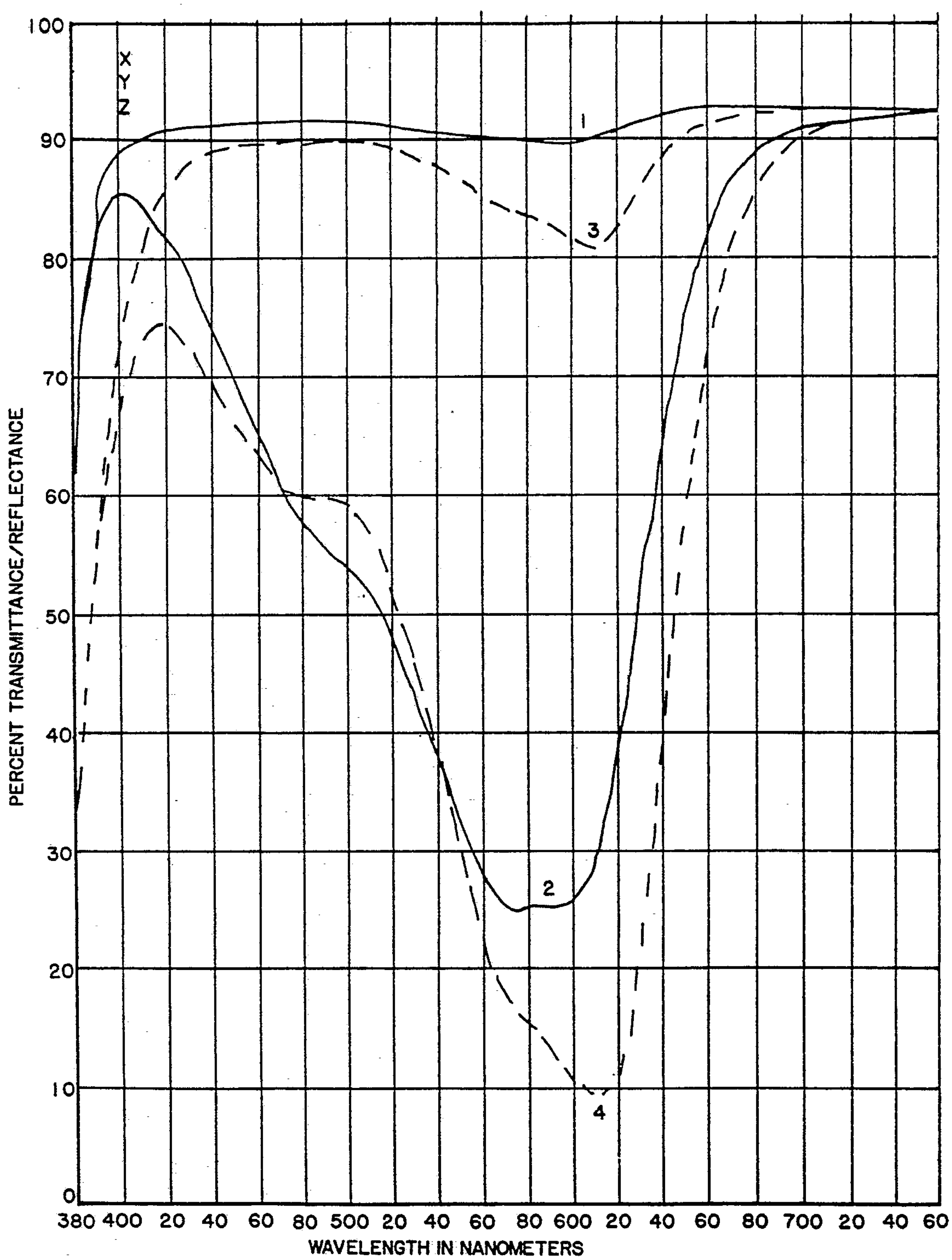
100
90
80
70
60
50
40
30
20
10
0
X
Y
Z
1
2
3
4
PERCENT TRANSMITTANCE/REFLECTANCE
380 400 20 40 60 80 500 20 40 60 80 600 20 40 60 80 700 20 40 60
WAVELENGTH IN NANOMETERS

Fig. 1

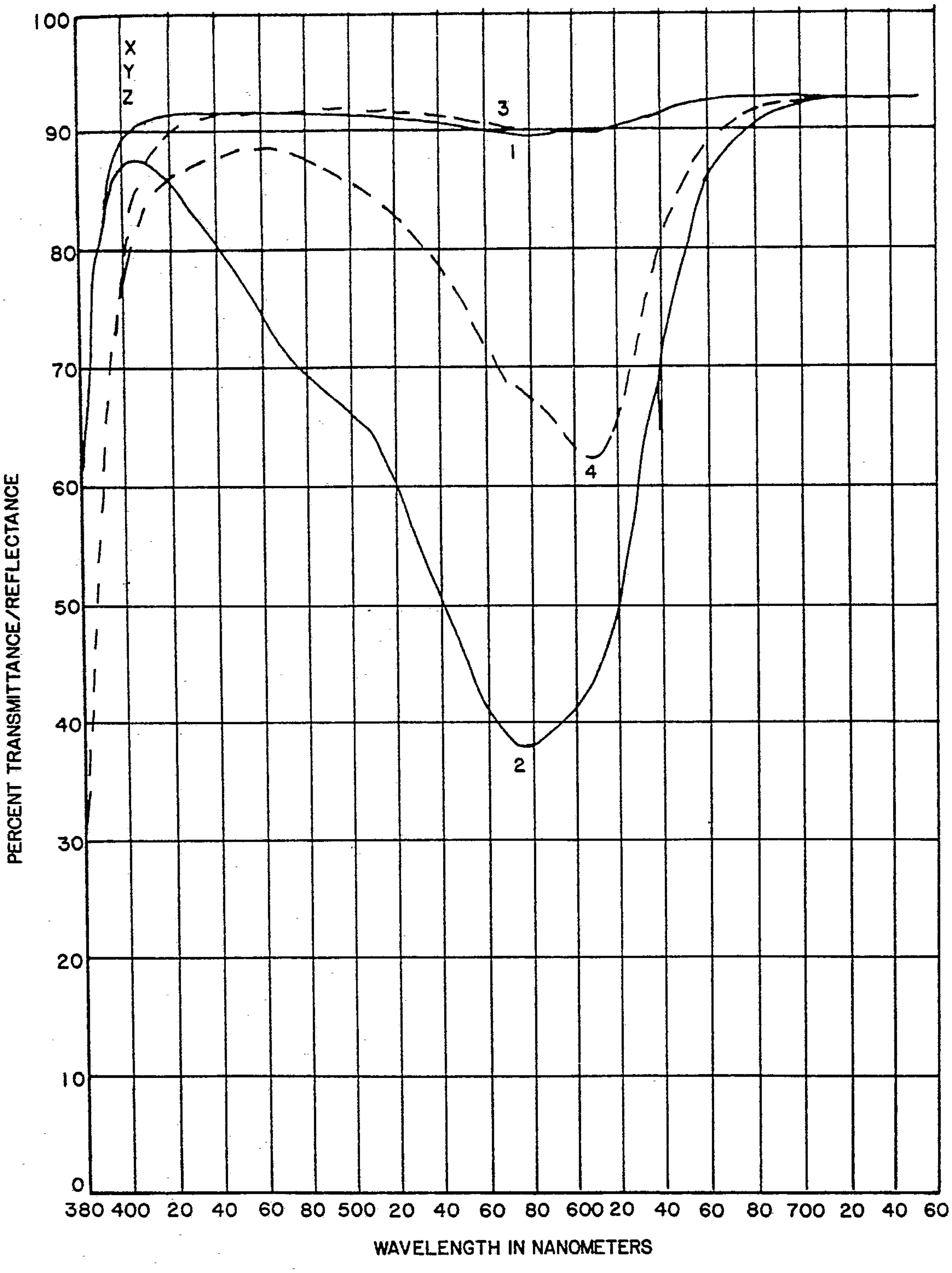
PERCENT TRANSMITTANCE/REFLECTANCE
100
90
80
70
60
50
40
30
20
10
0
X
Y
Z
3
1
4
2
380 400 20 40 60 80 500 20 40 60 80 600 20 40 60 80 700 20 40 60
WAVELENGTH IN NANOMETERS

Fig. 2